# EUROPÄISCHE PATENTSCHRIFT

(11) **EP 2 158 808 B1**
(45) Veröffentlichungstag und Bekanntmachung des Hinweises auf die Patenterteilung: **10.11.2010**
(21) Anmeldenummer: 08163189.7
(22) Anmeldetag: 28.08.2008
(51) Int. Cl.: A01G 31/02, A01K 63/00

(54) **Aquaponikanlage zur Gemüse-und Fischproduktion**
Aquaponics facility for producing vegetables and fish
Installation aquaponique destinée à la production de légumes et de poissons

(43) Veröffentlichungstag der Anmeldung: 03.03.2010
(73) Patentinhaber: Forschungsverbund Berlin E.V., 12489 Berlin (DE)
(72) Erfinder: Kloas, Werner, 12589, Berlin (DE); Rennert, Bernhard, 12555, Berlin (DE); Van Ballegooy, Christoph, 10317, Berlin (DE); Drews, Manfred, 14979, Großbeeren (DE)
(74) Vertreter: Hengelhaupt, Jürgen

(56) Entgegenhaltungen:
- EP-A- 1 112 680
- DD-A1- 240 327
- JP-A- 2001 190 166
- US-A- 5 046 451

## Beschreibung

### Technisches Gebiet

Die vorliegende Erfindung betrifft eine Aquaponikanlage mit geschlossenem Wasserkreislauf, ein Verfahren zur Herstellung von Aquaponik-Produkten und die Verwendung einer Aquaponikanlage.

### Technologischer Hintergrund und Stand der Technik

Als Aquakultur wird die kontrollierte Aufzucht von aquatischen Organismen, wie z. B. Fischen, Krebsen, Muscheln oder Wasserpflanzen, wie z. B. Algen bezeichnet. Die Aquakultur und die Aquakulturtechnologie sind ein weltweit stark wachsender Markt. Gegenwärtig werden ca. 29% des Weltfischereiertrages durch Produkte aus der Aquakultur gedeckt.

Ein Problem der Aquakultur ist, dass das Wasser im Laufe der Aufzucht durch Stoffwechselprodukte der Tiere, beispielsweise der Fische, und/oder durch Rückstände der zugesetzten Futtermittel belastet wird und gereinigt werden muss, um die Produktivität der Zucht nicht zu gefährden.

In sogenannten offenen Aquakulturanlagen wird dies durch Ersetzen des Wassers mit Frischwasser und Ableiten des verwendeten Wassers in die Umgebung erreicht. Dadurch wird die Umwelt stark belastet, vorhandene natürliche Gewässer können eutrophiert und sogar hypertrophiert werden, außerdem ist der Wasserverbrauch solcher Anlagen sehr hoch. Damit steigen die Kosten solcher Anlagen und sie können nur an Orten mit ausreichendem Wasservorkommen betrieben werden.

Um diese Nachteile so gering wie möglich zu halten, sind Aquakulturanlagen mit geschlossenem Wasserkreislauf entwickelt worden, bei denen das benutzte, belastete Wasser durch eine kombinierte mechanisch-biologische Wasserreinigung aufbereitet und der Fischzucht zurückgeführt wird.

Bei der biologischen Reinigung kommen verschiedene biologische Filter zum Einsatz. In diesen Filtern werden die von den Fischen ausgeschiedenen Stickstoffverbindungen, besonders Ammonium bzw. Ammoniak, durch bakterielle Nitrifikation zu Nitrat oxidiert. Der Prozess der Nitrifikation führt in einem geschlossenen Kreislaufsystem zu einem pH-Abfall bei gleichzeitiger Nitratakkumulation im aufbereiteten Wasser. Diesem Vorgang kann man entweder durch den Einsatz einer Denitrifikationsstufe oder durch erhöhte Frischwasserzugabe begegnen. In beiden Fällen wird der Stickstoff ungenutzt als Emission in die Umwelt freigesetzt. Der Stickstoff, speziell der Nitratstickstoff, ist aber sehr gut für die Nährstoffversorgung von Pflanzen geeignet. Deshalb wurden schon in der Vergangenheit Versuche zur kombinierten Fisch- und Pflanzenproduktion mit dem Ziel einer besseren Nährstoffausnutzung und Wasserreinigung durchgeführt. Es entstanden sogenannte Aquaponikanlagen, wobei in eine geschlossene Aquakulturanlage eine Hydroponikkultur (oder Hydrokultur) integriert wurde, die das Nitrat-haltige Wasser nach der Nitrifikation aufnimmt. Dabei wird das Nitrat-haltige Abwasser aus der Aquakultur in einer Hydroponikkultur Pflanzen als Nährlösung angeboten. Danach wird das Wasser, welches das nicht von den Pflanzen aufgenommene Nitrat enthält, wieder der Aquakultur zugeführt. Die Pflanzen wirken dabei als Nitratabnehmer. Geeignete Aquaponikanlagen sind z. B. in der Patentschrift DD 240 327 A1 und von Rennert und Drews, 1989 (B. Rennert, M. Drews; Eine Möglichkeit der kombinierten Fisch- und Gemüseproduktion in Gewächshäusern; Fortschr. Fisch.wiss. 8 (1989): 19-27) beschrieben worden.

Ein Problem dieser Anlagen liegt darin, dass die Ansprüche der Zuchttiere der Aquakultur und der Pflanzen der Hydroponikkultur an das Wasser unterschiedlich sind. Während Pflanzen im Wurzelbereich einen pH-Wert kleiner als 6 benötigen, um produktiv wachsen zu können, sind Fische auf einen pH-Wert von größer als 6 angewiesen, um rentabel produziert werden zu können. Während das Nitrat-haltige Wasser, so wie es aus dem biologischen Filter austritt, den pH-Werterfordernissen der Pflanzen entspricht, enthält das Wasser, welches in die Aquakultur zurückgeführt werden soll, noch zuviel Restnitrat und weist nicht den erforderlichen, für die Fische günstigen pH-Wert auf. In den herkömmlichen Aquaponik-Systemen wurde der damit notwendige pH-Wertausgleich im Wesentlichen durch Zufuhr von Frischwasser erreicht. Insgesamt lag die benötigte Wassererneuerung in der Aquaponikanlage zur Einstellung des pH-Wertes und zur Verhinderung der Akkumulation von Nitrat im aufbereiteten Wasser durchschnittlich bei 20 bis 25% des Gesamtwasservolumens der Anlage pro Tag (Rennert und Drews 1989). Bei einem solch hohen Wasserverbrauch sind diese Aquaponiksysteme nur mit hochpreisigen Fischen rentabel zu betreiben und sind bzgl. der Lage der Anlage auf Gegenden mit ausreichender Wasserversorgung beschränkt.

Aufgabe der vorliegenden Erfindung war es einen oder mehrere der geschilderten Nachteile des Standes der Technik zu mindern oder zu überwinden.

### Erfindungsgemäße Lösung

Die Aufgabe wird durch Bereitstellung einer Aquaponikanlage mit geschlossenem Wasserkreislauf gelöst, umfassend mindestens eine Aquakultur-Einheit und mindestens eine Hydroponik-Einheit, dadurch gekennzeichnet, dass die Aquakultur-Einheit mindestens einen Wasserabfluss aufweist, der über ein Einwege-Ventil mit der Hydroponik-Einheit derart funktional verbunden ist, dass Wasser aus der Aquakultur-Einheit der Hydroponik-Einheit zuführbar ist, und die Hydroponik-Einheit mindestens eine Kühlfalle aufweist, wobei die mindestens eine Kühlfalle derart funktional mit der Aquakultur-Einheit verbunden ist, dass das gewonnene Wasser der mindestens einen Kühlfalle der Aquakultur-Einheit zuführbar ist.

Bei der erfindungsgemäßen Aquaponikanlage fließt das benutzte Wasser der Aquakultur-Einheit durch einen Wasserabfluss über ein Einwege-Ventil der Hydroponik-Einheit zu. Dort wird das Wasser verwendet, um die Pflanzen zu wässern und mit Nährstoffen zu versorgen. Die Pflanzen nehmen das Wasser und die Nährstoffe (u.a. Nitrat) auf, geben dann im Wege der Pflanzentranspiration Wasser ohne Nährstoffe (u.a. Nitrat) an die Luft in der Hydroponik-Einheit ab. Dieses Transpirationswasser der Pflanzen wird durch die Kühlfalle der Hydroponik-Einheit gesammelt und in die Aquakultur-Einheit zurückgeführt. Es entsteht ein geschlossener Wasserkreislauf in der Aquaponikanlage, bei dem die Pflanzen der Hydroponik-Einheit als natürlicher Filter für Nitrat und als natürliches Korrektiv für den pH-Wert des Wassers dienen. Die Pflanzen dienen dabei nicht nur als Abnehmer des im Wasser enthaltenen Nitrats, sondern als echter Nitrat-Filter in dem sie Transpirationswasser ab geben, dass im Wesentlichen frei von Nitrat ist. Ein Zuführen von Frischwasser zur Regulation des pH-Wertes oder der Nitratkonzentration im aufbereiteten Wasser vor der Rückführung in die Aquakultur-Einheit ist nicht mehr notwendig. Der erfindungsgemäßen Aquaponikanlage wird Wasser lediglich durch Entnahme von Biomasse in Form von Zuchttieren oder Pflanzenmaterial entzogen. Lediglich dieses muss bei Betrieb der Anlage zugeführt werden. Dadurch ist es möglich in einer bevorzugten Ausführungsform eine erfindungsgemäße Aquaponikanlage bereitzustellen, bei der die täglich notwendige Frischwasserzufuhr bei Betrieb der Anlage weniger als 5% des Gesamtwasservolumens der Anlage beträgt, besonders bevorzugt weniger als 3%.

Somit steht mit der erfindungsgemäßen Aquaponikanlage erstmals ein geschlossenes, nahezu emissionsfreies System zur Verfügung, in das im Wesentlichen nur Fischfutter und geringste Wassermengen eingebracht werden müssen. Dadurch kann die erfindungsgemäße Anlage umweltfreundlicher und kostengünstiger betrieben werden und eignet sich auch für den Einsatz in Gegenden, in denen weniger Wasser zur Verfügung steht.

Unter dem Begriff "Aquakultur-Einheit" ist i.S.d. Erfindung eine Anlage zu verstehen, die geeignet ist für die kontrollierte Aufzucht von aquatischen Organismen wie Fischen, Krebsen, Muscheln und Wasserpflanzen, wie beispielsweise Algen. Solche Aquakulturanlagen sind dem Fachmann bekannt und in der Literatur beschrieben.

In einer bevorzugten Ausführungsform weist die Aquakultur-Einheit mindestens einen Bereich für die Fischzucht, z. B. in Form eines Fischaufzuchttanks, -becken oder -rinnen, einen mechanischen Filter und einen biologischen Filter auf.

Geeignete mechanische Filter dienen dazu, Schwebstoffe, wie z. B. Kot und nicht aufgenommene Nahrungsbestandteile, aus dem belasteten Wasser aufzunehmen. Das im mechanischen Filter entstandene Sediment kann aus den mechanischen Filtern entnommen werden. Dem Fachmann sind geeignete mechanische Filter aus den Stand der Technik bekannt. Beispiele für geeignete mechanische Filter sind mechanische Filter wie Lamellenabscheider, Mikrosiebfilter und Absetzbecken. Bevorzugt werden Lamellenabscheider verwendet.

Geeignete biologische Filter dienen vorrangig der Nitrifikation, d.h. der Oxydation von Ammonium/Ammoniak über Nitrit zu Nitrat. Für die Nitrifikation werden entweder chemische Verfahren verwendet oder Mikroorganismen eingesetzt. Bevorzugt werden autotrophe Bakterien verwendet, besonders bevorzugt Bakterien der Gattungen *Nitrosomonas* und *Nitrobacter.* Daneben können geeignete Filter einen heterotrophen Bereich aufweisen, in welchem ein Kohlenstoffabbau erfolgt und Kohlendioxid freigesetzt wird. Bei der Verwendung von untergetauchten biologischen Filtern verbleibt ein grösserer Teil des entstandenen Kohlendioxids im Kreislaufwasser. Bevorzugt sind deshalb Biofilter in Form von Tropfkörpern, wobei hier das entstandene Kohlendioxid aus dem Tropfkörper abgasen kann und nicht im Kreislaufwasser verbleibt. In einer Ausführungsform der erfindungsgemäßen Aquaponikanlage wird das aus dem Tropfkörper abgegaste Kohlendioxid der Hydroponik-Einheit zugeführt. Dem Fachmann sind geeignete biologische Filter aus dem Stand der Technik bekannt. Bevorzugt wird ein Tropfkörper als biologischer Filter eingesetzt.

Die Aquakultur-Einheit ist über ein Einwege-Ventil mit der Hydroponik-Einheit funktional verbunden, sodass Wasser aus der Aquakultur-Einheit der Hydroponik-Einheit zugeführt werden kann. Geeignete Einwege-Ventile dienen dazu den Abfluss aus der Aquakultur-Einheit bzw. den Zufluss in die Hydroponik-Einheit zu steuern. Dabei ist das erfindungsgemäß geeignete Einwege-Ventil so beschaffen, dass es eine Wasserbewegung im Wesentlichen nur in eine Richtung, aus der Aquakultur-Einheit in die Hydroponik-Einheit zulässt. Geeignete Einwege-Ventile sind regel- bzw. steuerbar. Die Regelung bzw. Steuerung kann manuell oder automatisch, ggf. gesteuert durch einen Computer erfolgen. Grundsätzlich ist jedes Ventil geeignet, dass eine Wasserbewegung im Wesentlichen nur in eine Richtung zulässt und regelbar ist. Dem Fachmann sind geeignete Einwege-Ventile aus dem Stand der Technik bekannt. Dem Fachmann ist bekannt, dass die Kapazität des Einwege-Ventils an die Gesamtdimension der Aquaponikanlage angepasst sein soll, um einen reibungslosen Betrieb der Anlage zu ermöglichen. In einer bevorzugten Ausführungsform weist die Aquakultur-Einheit mehr als ein Einwege-Ventil auf. Unter Berücksichtigung der Gesamtdimension der Anlage kann der Fachmann so, ohne großen Aufwand, die geeignete Kapazität an Wasserab- bzw. -zufluss bereitstellen und einen besonders günstigen Betrieb der Anlage gewährleisten. In einer bevorzugten Ausführungsform handelt es sich bei dem Einwege-Ventil um ein Magnetventil. Das Einwege-Ventil kann z. B. von einem Schwimmschalter im Nährstoffbecken der Hydroponik-Einheit angesteuert werden. D.h. wenn der Wasserstand im Nährlösungsbecken z. B. durch die Wasseraufnahme der Pflanzen sinkt, öffnet das Einwege-Ventil und Wasser aus dem Fischkreislauf wird in die Hydroponik-Einheit nachgespeist. Ein Wasserfluss in die entgegengesetzte Richtung ist dabei nicht möglich.

Unter dem Begriff "Hydroponik-Einheit" ist i.S.d. Erfindung eine Anlage zu verstehen, die geeignet ist zur Pflanzenzucht und -haltung, bei der die Pflanzen in einem anorganischen Substrat oder ohne Substrat gemäß der sogenannten Nährfilmtechnik (NFT, siehe z. B. Graves, C.J. (1993): The nutrient film techique. Horticult Rev, 5, 1-44) wurzeln, anstatt in einem, organische Bestandteile enthaltenden Boden. Die Ernährung der Pflanzen erfolgt dabei über eine wässrige Lösung anorganischer Nährsalze. Die Hydroponik-Einheit gemäß der Erfindung umfasst definitionsgemäß immer mindestens ein Gewächshaus in dem sich die Pflanzenkultur befindet. In einer Ausführungsform der Aquaponikanlage, in der sowohl die Aquakultur-Einheit, als auch die Hydroponik-Einheit in einem gemeinsamen Gewächshaus angeordnet sind, ist das Gewächshaus der Hydroponik-Einheit gleichzeitig das Gewächshaus, welches die Aquakultur- und die Hydroponik-Einheit umfasst. In einer bevorzugten Ausführungsform der Aquaponikanlage weist die Hydroponik-Einrichtung mindestens einen Bereich für die Zubereitung und/oder Speicherung der Nährlösung, z. B. einen Nährlösungstank in dem ggf. weitere Nährstoffe oder Zusätze beigemischt werden können, und einen Bereich für die Pflanzenkultur auf. Dem Fachmann sind geeignete Hydroponik-Einheiten aus dem Stand der Technik bekannt, beispielsweise aus Rennert und Drews (1989) oder der Patentschrift DD 240 327 A1.

Die erfindungsgemäße Aquaponikanlage umfasst eine Hydroponik-Einrichtung, die mindestens eine Kühlfalle aufweist. Geeignete Kühlfallen dienen dazu, Wasser aus der Luft der Hydroponik-Einheit oder dem Luftraum der kombinierten Hydroponik- und Aquakultur-Einheit zu kondensieren und zu sammeln. Entsprechende Kühlfallen und Kühlfallentechnik sind dem Fachmann aus dem Stand der Technik bekannt. Grundsätzlich eignet sich jede Kühlfalle für den Einsatz in der erfindungsgemäßen Aquaponikanlage. Dem Fachmann ist bekannt, dass die Kapazität der Kühlfalle bzw. der Kühlfallen an die Gesamtdimension der Aquaponikanlage angepasst sein soll, um einen reibungslosen Betrieb der Anlage zu ermöglichen. In einer bevorzugten Ausführungsform weist die Hydroponik-Einheit mehr als eine Kühlfalle auf, wobei die Kühlfallen nebeneinander und/oder in Reihe angeordnet sein können. Unter Berücksichtigung der Gesamtdimension der Anlage kann der Fachmann so, ohne großen Aufwand, die geeignete Kapazität an Kühlfallenaktivität bereitstellen und einen besonders günstigen Betrieb der Anlage gewährleisten.

In einer bevorzugten Ausführungsform der Aquaponikanlage ist sowohl die Aquakultur-Einheit, als auch die Hydroponik-Einheit in einem gemeinsamen Gewächshaus funktional angeordnet, sodass ein gemeinsamer, zusammenhängender Luftraum entsteht, der mit der mindestens einen Kühlfalle der Hydroponik-Einheit verbunden ist. Bei dieser Anordnung kann die Kühlfalle nicht nur das Transpirationswasser der Pflanzen aus dem Luftraum zurückgewinnen, sondern auch das Verdunstungswasser der Aquakultur-Einheit kondensieren und sammeln. Der Wasserverlust beim Betrieb der Anlage wird weiter vermindert gegenüber bekannten Anlagen aus dem Stand der Technik.

Die erfindungsgemäße Aquaponikanlage kann zusätzlich eine Photovoltaikanlage aufweisen. Dabei ist die Photovoltaikanlage so angebracht, dass diese Sonnenenergie in geeigneter Weise aufnehmen und in elektrische Energie umwandeln kann. Geeignete Photovoltaikanlagen kann der Fachmann dem Stand der Technik entnehmen. Die Photovoltaikanlage kann, je nach Standort der Anlage und Platzangebot, auf dem Dach des Gewächshauses oder als Freiflächenanlage angebracht sein. Die geeignete Photovoltaikanlage stellt elektrischen Strom bereit für den Betrieb der mindestens einen Kühlfalle der Hydroponik-Einrichtung. Bei der Auswahl einer geeigneten Photovoltaikanlage zieht der Fachmann die Gesamtdimension der Aquaponikanlage in Betracht und richtet sich bei der Größe der Photovoltaikanlage nach dem zu erwartenden Stromverbrauch der Kühlfalle(n) der Hydroponik-Einheit. Ggf. kann überschüssige Energie der Photovoltaikanlage zur Temperaturregulation des Wassers der Aquakultur-Einheit verwendet werden.

Die erfindungsgemäße Aquaponikanlage kann eine Biogasanlage aufweisen. Geeignete Biogasanlagen sind in der Lage aus Biomasse Biogas zu erzeugen und aus dem gewonnen Biogas elektrische Energie zu gewinnen. Die Biogasanlage der erfindungsgemäßen Aquaponikanlage kann mit dem Sediment aus dem mechanischen Filter der Aquakultur-Einheit sowie mit Fisch- und Pflanzenabfällen betrieben werden. Die elektrische Energie der Biogasanlage kann sowohl zum Betrieb der Kühlfalle(n) der Hydroponik-Einheit, als auch zum Betrieb der Temperaturregulation für das Wasser der Aquakultur-Einheit verwendet werden.

In einer besonders bevorzugten Ausführungsform der Aquaponikanlage wird die Aquakultur-Einheit mit fischmehl- und/oder fischölfreiem Futter betrieben. Dabei wird bevorzugt Futter eingesetzt, bei dem das Fischmehl durch Fliegenmadenmehl und das Fischöl durch Pflanzenöl vollständig substituiert wurde.

In einer weiteren bevorzugten Ausführungsform wird die Aquakultur-Einheit mit Fischen, bevorzugt Tilapien, besonders bevorzugt mit *Oreochromis niloticus* betrieben. Diese Fische eignen sich besonders gut für die Aquakultur, da sie sich zu jeder Jahreszeit leicht vermehren lassen, resistent gegenüber erhöhten Wassertemperaturen (von über 30°C) sind, wie sie in Sommermonaten in Gewächshäusern auftreten können, und daneben noch grätenarm und schmackhaft sind.

Bevorzugt wird die Hydroponik-Einheit der erfindungsgemäßen Aquaponikanlage mit Gemüsepflanzen betrieben, besonders bevorzugt mit Tomaten (z. B. *Solanum Iycopersicum)* und/oder Gurken (z. B. *Cucumis sativus).* Die Hydroponik-Einheit der erfindungsgemäßen Aquaponikanlage kann ebenfalls mit anderen Pflanzen als Gemüsepflanzen betrieben werden. Besonders geeignet sind in jedem Fall solche Pflanzen, die sich durch eine besonders hohe Nitrataufnahme- und Verarbeitungskapazität auszeichnen, wie z. B. *Ceratophyllum demersum* (Gemeines Hornkraut), Basilikum *(Ocimum basilicum*), Okra *(Abelmoschus esculentus)* und verschiedene Salate.

Dem Fachmann ist bewusst, dass, um mit der erfindungsgemäßen Aquaponikanlage optimale Ergebnisse erzielen zu können, verschiedene Faktoren bei der Dimensionierung der Anlage in Betracht gezogen werden müssen. So hat die Wahl der Fischart und die Menge der Fische der Aquakultur-Einheit zusammen mit dem Gesamtwasservolumen der Aquaponikanlage einen bestimmenden Einfluss darauf, wie die Hydroponik-Einheit betrieben und dimensioniert sein muss, um ein besonders günstiges Betriebsergebnis zu erreichen. Auch andere Faktoren, wie z. B. die Temperatur des Wassers und der Umgebung, die durchschnittliche Lichteinstrahlungsdauer und Lichtintensität pro Zeit sind zu berücksichtigen. All diese Faktoren werden nicht nur von der Wahl der Fische der Aquaponik-Einheit und der Wahl der Pflanzen der Hydroponik-Einheit bestimmt, sondern hängen auch von der Wahl des Standorts und der Gesamtdimension der Anlage ab. Der Fachmann hat keine Schwierigkeiten bei der Planung und beim Bau der erfindungsgemäßen Aquaponikanlage die beschriebenen Einflussfaktoren einzubeziehen und zu einer funktionsfähigen Aquaponikanlage zu gelangen, die die beschriebenen erfindungsgemäßen Vorteile aufweist. So kann z. B. der Betrieb der Aquakultur-Einheit mit einer größeren Fischmenge dadurch aufgefangen werden, dass die Hydroponik-Einheit mit Pflanzen mit einer besonders hohen Nitrataufnahme- und Verarbeitungskapazität betrieben wird.

In einem weiteren Aspekt betrifft die Erfindung ein Verfahren zum Betrieb einer Aquaponikanlage, welches dadurch gekennzeichnet ist, dass:
a) Wasser aus einer Aquakultur-Einheit über einen Wasserabfluss mit einem Einwege-Ventil einer Hydroponik-Einheit zugeführt wird;
b) das Wasser von Pflanzen der Hydroponik-Einheit aufgenommen und durch Pflanzentranspiration an die Atmosphäre der Hydroponik-Einheit abgegeben wird;
c) das Wasser aus der Atmosphäre der Hydroponik-Einheit durch Kondensation gesammelt wird; und
d) das gesammelte Wasser in die Aquakultur-Einheit zurückgeführt wird.

Die Kondensation des Wassers aus der Atmosphäre der Hydroponik-Einheit kann durch jedes geeignete Verfahren erfolgen, bevorzugt werden eine oder mehrere Kühlfallen verwendet.

In einer bevorzugten Ausgestaltung des Verfahrens wird die Aquakultur-Einheit mit fischmehl- und/oder fischölfreiem Futter betrieben.

In einer Ausgestaltung des erfindungsgemäßen Verfahrens wird die Aquakultur-Einheit mit Tilapien, bevorzugt mit *Oreochromis niloticus* betrieben.

In einer weiteren Ausgestaltung des erfindungsgemäßen Verfahrens wird die Hydroponik-Einheit mit Gemüsepflanzen, bevorzugt mit Tomaten und/oder Gurken betrieben.

Die Erfindung bezieht sich auch auf ein Verfahren zur Herstellung von Aquakultur-Produkten wie z. B. Fisch, Krebsen, Muscheln oder Wasserpflanzen, wie z. B. Algen und/oder von Hydroponik-Produkten, z. B. Gemüse, wie Tomaten und/oder Gurken, wobei eine erfindungsgemäße Aquaponikanlage zum Einsatz kommt.

Des Weiteren bezieht sich die Erfindung auf eine Verwendung einer erfindungsgemäßen Aquaponikanlage zur Herstellung von Aquakultur- und/oder Hydroponik-Produkten.

### Figuren

- Fig. 1: zeigt eine Ausführungsform der erfindungsgemäßen Aquaponikanlage
- Fig. 2: zeigt eine weitere Ausführungsform der erfindungsgemäßen Aquaponikanlage, umfassend ein gemeinsames Gewächshaus mit einer Aquakultur- und Hydroponik-Einheit, sowie einer Klimaanlage mit Kühlfalle, einer Photovoltaikanlage und einer Biogasanlage.

### Ausführungsbeispiele

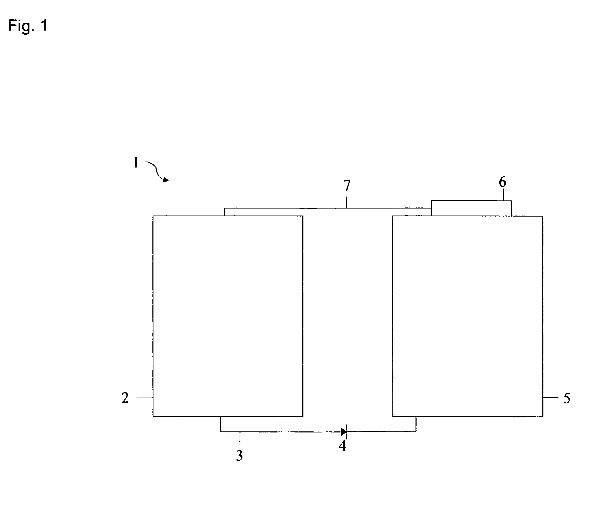

In Fig. 1 ist ein Ausführungsbeispiel einer erfindungsgemäßen Aquaponikanlage 1 dargestellt. Diese umfasst eine Aquakultur-Einheit 2, die über einen Wasserabfluss 3 mit einem Einwege-Ventil 4 mit einer Hydroponik-Einheit 5 verbunden ist. Die Hydroponik-Einheit 5 weist eine Kühlfalle 6 auf, die über einen Rückfluss 7 mit der Aquakultur-Einheit 2 verbunden ist. Das benutzte Wasser aus der Aquakultur-Einheit 2 wird über den Wasserabfluss 3 durch das Einwege-Ventil 4 der Hydroponik-Einheit 5 zugeleitet. Dort wird das Wasser einschließlich der Nitrate und anderen Nährstoffe von den Pflanzen aufgenommen. Die Pflanzen geben Wasser in Form von Pflanzentranspiration wieder an die Umgebungsluft in der Hydroponik-Einheit 5 ab. Dieses Wasser wird durch die Kühlfalle 6 aus der Umgebungsluft kondensiert und gesammelt. Schließlich wird das aufbereitete Wasser aus der Kühlfalle 6 über den Rückfluss 7 in die Aquakultur-Einheit 2 zurückgeführt, der Wasserkreislauf ist geschlossen.

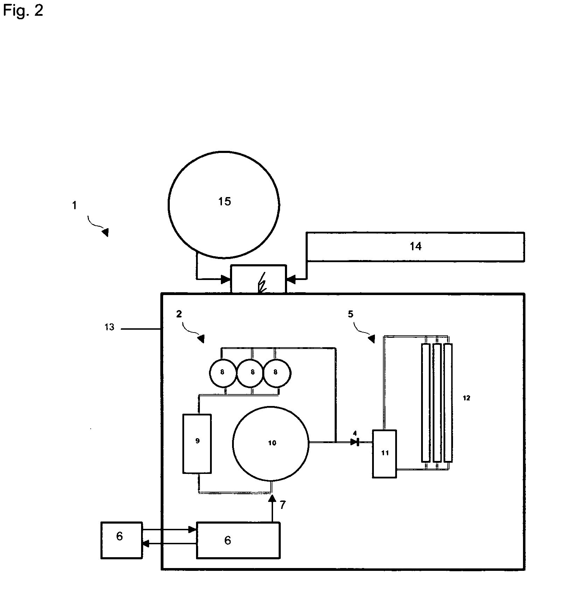

In Fig. 2 ist ein weiteres Ausführungsbeispiel einer erfindungsgemäßen Aquaponikanlage 1 dargestellt. Zusätzlich zu der Ausführungsform aus Fig. 1 sind nun weitere Bestandteile der Aquakultur-Einheit und der Hydroponik-Einheit wiedergegeben. In dieser Ausführungsform befinden sich Aquakultur- 2 und Hydroponik-Einheit 5 in einem gemeinsamen Gewächshaus 13. Das benutzte Wasser aus den Fischaufzuchttanks 8 wird einem mechanischen Filter 9 zugeleitet, welcher Schwebstoffe mechanisch aus dem Wasser sedimentiert. Anschließend wird das gereinigte Wasser aus dem mechanischen Filter 9 dem biologischen Filter 10 zugeführt. In dem biologischen Filter findet die Nitrifikation statt und es wird Kohlendioxid gebildet. Das nun Nitrat enthaltende Wasser wird z. B. bei Bedarf aus der Aquakultur-Einheit 2 über das Einwege-Ventil 4 in die Hydroponik-Einheit 5 überführt und gelangt dort in das Nährlösungsbecken 11 in dem ggf. weitere Zusätze oder Nährstoffe beigemengt werden können. Anschließend wird das Wasser den Pflanzrinnen 12 zugeführt. Dort wird das Wasser einschließlich der Nitrate und anderen Nährstoffe von den Pflanzen aufgenommen. Nichtaufgenommenes Wasser einschließlich nichtaufgenommener Nitrate wird in das Nährlösungsbecken 11 zurückgeführt. Die Pflanzen geben Wasser in Form von Pflanzentranspiration wieder an die Umgebungsluft in der Hydroponik-Einheit 5 ab. Dieses Wasser wird durch die Kühlfalle 6 aus der Umgebungsluft kondensiert und gesammelt. Schließlich wird das aufbereitete Wasser aus der Kühlfalle 6 über den Rückfluss 7 in die Aquakultur-Einheit 2 zurückgeführt, der Wasserkreislauf ist geschlossen. Die Energieversorgung wird bei dieser Ausführungsform gemeinsam über eine Photovoltaikanlage 14 und eine Biogasanlage 15 gewährleistet.

Liste der Bezugszeichen
- 1: Aquaponikanlage
- 2: Aquakultur-Einheit
- 3: Wasserabfluß
- 4: Einwege-Ventil
- 5: Hydroponik-Einheit
- 6: Kühlfalle, ggf. gekoppelt mit Klimaanlage
- 7: Rückfluß
- 8: Fischaufzuchttank
- 9: mechanischer Filter
- 10: biologischer Filter
- 11 1: Nährlösungsbecken
- 12: Pflanzrinnen
- 13: Gewächshaus
- 14: Photovoltaikanlage
- 15: Biogasanlage

## Patentansprüche

1. Aquaponikanlage (1) mit geschlossenem Wasserkreislauf umfassend mindestens eine Aquakultur-Einheit (2) und mindestens eine Hydroponik-Einheit (5), wobei die Aquakultur-Einheit (2) mindestens einen Wasserabfluss (3) aufweist,
**dadurch gekennzeichnet, dass** der Wasserabfluss über ein Einwege-Ventil (4) mit der Hydroponik-Einheit (5) derart funktional verbunden ist, dass Wasser aus der Aquakultur-Einheit (2) der Hydroponik-Einheit (5) zuführbar ist, und
die Hydroponik-Einheit (5) mindestens eine Kühlfalle (6) aufweist, wobei die mindestens eine Kühlfalle (6) derart funktional mit der Aquakultur-Einheit (2) verbunden ist, dass das gewonnene Wasser der mindestens einen Kühlfalle (6) der Aquakultur-Einheit (2) zuführbar ist.

2. Aquaponikanlage (1) nach Anspruch 1, **dadurch gekennzeichnet, dass** die Hydroponik-Einheit (5) mehr als eine Kühlfalle (6) aufweist, wobei die Kühlfallen (6) nebeneinander und/oder in Reihe angeordnet sind.

3. Aquaponikanlage (1) nach einem der vorergehenden Ansprüche, **dadurch gekennzeichnet, dass** das Einwege-Ventil (4) manuell oder automatisch regelbar ausgebildet ist.

4. Aquaponikanlage (1) nach einem der vorergehenden Ansprüche, **dadurch gekennzeichnet, dass** die Hydroponik-Einheit (5) mindestens einen Bereich für die Zubereitung und/oder Speicherung der Nährlösung (11) und einen Bereich für die Pflanzenkultur (12) aufweist.

5. Aquaponikanlage (1) nach einem der vorhergehenden Ansprüche, **dadurch gekennzeichnet, dass** die Aquakultur-Einheit (2) mindestens einen Bereich für die Fischaufzucht (8), einen mechanischen Filter (9) und einen biologischen Filter (10) aufweist

6. Aquaponikanlage (1) nach einem der vorergehenden Ansprüche, **dadurch gekennzeichnet, dass** sowohl die Aquakultur-Einheit (2), als auch die Hydroponik-Einheit (5) in einem gemeinsamen Gewächshaus derart funktional angeordnet sind, dass ein gemeinsamer, zusammenhängender Luftraum entsteht, der mit der mindestens einen Kühlfalle (6) der Hydroponik-Einheit (5) verbunden ist.

7. Aquaponikanlage (1) nach einem der vorergehenden Ansprüche, **dadurch gekennzeichnet, dass** die tägliche Frischwasserzufuhr bei Betrieb der Anlage weniger als 5% des Gesamtwasservolumens der Anlage beträgt.

8. Aquaponikanlage (1) nach einem der vorergehenden Ansprüche, **dadurch gekennzeichnet, dass** die Aquaponikanlage (1) zusätzlich eine Photovoltaikanlage aufweist.

9. Aquaponikanlage (1) nach einem der vorergehenden Ansprüche, **dadurch gekennzeichnet, dass** die Aquaponikanlage (1) eine Biogasanlage aufweist.

10. Verfahren zum Betrieb einer Aquaponikanlage (1), **dadurch gekennzeichnet, dass**
a) Wasser aus einer Aquakultur-Einheit (2) über einen Wasserabfluss (3) mit einem Einwege-Ventil (4) einer Hydroponik-Einheit (5) zugeführt wird;
b) das Wasser von Pflanzen der Hydroponik-Einheit (5) aufgenommen und durch Pflanzentranspiration an die Atmosphäre der Hydrponik-Einheit abgegeben wird;
c) das Wasser aus der Atmosphäre der Hydroponik-Einheit (5) durch Kondensation gesammelt wird; und
d) das gesammelte Wasser in die Aquakultur-Einheit (2) zurückgeführt wird.

11. Verfahren nach Anspruch 10, **dadurch gekennzeichnet, dass** die Aquakultur-Einheit (2) mit fischmehl- und/oder fischölfreiem Futter betrieben wird.

12. Verfahren nach einem der Ansprüche 10 oder 11, **dadurch gekennzeichnet, dass** die Aquakultur-Einheit (2) mit Tilapien, bevorzugt mit *Oreochromis niloticus* betrieben wird.

13. Verfahren nach einem der Ansprüche 10 bis 12, **dadurch gekennzeichnet, dass** die Hydroponik-Einheit (5) mit Gemüsepflanzen, bevorzugt mit Tomaten und/oder Gurken betrieben wird.

14. Verfahren zur Herstellung von Aquakultur-Produkten, **dadurch gekennzeichnet, dass** eine Aquaponikanlage (1) nach einem der Ansprüche 1 bis 9 eingesetzt wird.

15. Verfahren zur Herstellung von Hydroponik-Produkten, **dadurch gekennzeichnet, dass** eine Aquaponikanlage (1) nach einem der Ansprüche 1 bis 9 eingesetzt wird.

16. Verwendung einer Aquaponikanlage (1) nach einem der Ansprüche 1 bis 9 zur Gewinnung von Aquakultur- und/oder Hydroponik-Produkten.

## Claims

1. Aquaponic facility (1) with closed water circulation, comprising at least one aquaculture unit (2) and at least one hydroponic unit (5), in which the aquaculture unit (2) has at least one water outlet (3),
**characterized in that**
the water outlet (3) is functionally connected via a one-way valve (4) with the hydroponic unit (5) such that water from the aquaculture unit (2) can be supplied to the hydroponic unit (5),
and
the hydroponic unit (5) has at least one cold trap (6), wherein the at least one cold trap (6) is functionally connected with the aquaculture unit (2) in such a way that the water obtained from the at least one cold trap (6) can be supplied to the aquaculture unit (2).

2. Aquaponic facility (1) according to claim 1, **characterized in that** the hydroponic unit (5) has more than one cold trap (6), wherein the cold traps (6) are arranged side-by-side and/or sequentially.

3. Aquaponic facility (1) according to one of the preceding claims, **characterized in that** the one-way valve (4) is configured for manual or automatic control.

4. Aquaponic facility (1) according to one of the preceding claims, **characterized in that** the hydroponic unit (5) has at least one area for the preparation and/or storage of the nutrient solution (11) and one area for horticulture.

5. Aquaponic facility (1) according to one of the preceding claims, **characterized in that** the aquaculture unit (2) has at least one area for pisciculture (8), a mechanical filter (9) and a biological filter (10).

6. Aquaponic facility (1) according to one of the preceding claims, **characterized in that** the aquaculture unit (2) and the hydroponic unit (5) are functionally arranged in a common greenhouse such that a common continuous air space is created which is connected with the at least one cold trap (6) of the hydroponic unit (5).

7. Aquaponic facility (1) according to one of the preceding claims, **characterized in that** the daily supply of fresh water during operation of the facility is less than 5% of the total water volume of the facility.

8. Aquaponic facility (1) according to one of the preceding claims, **characterized in that** the aquaponic facility (1) additionally comprises a photovoltaic system.

9. Aquaponic facility (1) according to one of the preceding claims, **characterized in that** the aquaponic facility (1) comprises a biogas system.

10. Method for operating an aquaponic facility (1), **characterized in that**
a) water from an aquaculture unit (2) is supplied to a hydroponic unit (5) via a water outlet (3) having a one-way valve (4);
b) the water is absorbed by plants of the hydroponic unit (5) and released through plant transpiration into the atmosphere of the hydroponic unit;
c) the water from the atmosphere of the hydroponic unit (5) is collected through condensation; and
d) the collected water is returned to the aquaculture unit (2).

11. Method according to claim 10, **characterized in that** the aquaculture unit (2) is operated with fish meal and/or fish-oil-free feedstock.

12. Method according to one of the claims 10 or 11, **characterized in that** the aquaculture unit (2) is operated with tilapias, preferably with *oreochromis niloticus.*

13. Method according to one of the claims 10 to 12, **characterized in that** the hydroponic unit (5) is operated with vegetable plants, preferably with tomatoes and/or cucumbers.

14. Method for producing aquaculture products, **characterized in that** an aquaponic facility (1) according to one of the claims 1 to 9 is used.

15. Method for producing hydroponic products, **characterized in that** an aquaponic facility (1) according to one of the claims 1 to 9 is used.

16. Use of an aquaponic facility (1) according to one of the claims 1 to 9 for producing aquaculture and/or hydroponic products.

## Revendications

1. Installation aquaponique (1) avec circuit d'eau fermé comprenant au moins une unité d'aquaculture (2) et au moins une unité hydroponique (5), l'unité d'aquaculture (2) présentant au moins une évacuation d'eau (3), **caractérisée en ce que** l'évacuation d'eau est raccordée de façon fonctionnelle, par le biais d'une vanne à une voie (4), à l'unité hydroponique (5) de sorte que l'eau peut être conduite de l'unité d'aquaculture (2) à l'unité hydroponique (5),
et
l'unité hydroponique (5) présente au moins un piège à froid (6), le piège à froid (6), au moins au nombre de un, étant raccordé de façon fonctionnelle à l'unité d'aquaculture (2) de sorte que l'eau acquise du piège à froid (6), au moins au nombre de un, peut être conduite à l'unité d'aquaculture (2).

2. Installation aquaponique (1) selon la revendication 1, **caractérisée en ce que** l'unité hydroponique (5) présente plus d'un piège à froid (6), les pièges à froid (6) étant disposés les uns à côté des autres et/ou en série.

3. Installation aquaponique (1) selon une des revendications précédentes, **caractérisée en ce que** la vanne à une voie (4) est constituée de façon à pouvoir être réglée manuellement ou automatiquement.

4. Installation aquaponique (1) selon une des revendications précédentes, **caractérisée en ce que** l'unité hydroponique (5) présente au moins une zone pour la préparation et/ou le stockage de la solution nutritive (11) et une zone pour la culture des plantes (12).

5. Installation aquaponique (1) selon une des revendications précédentes, **caractérisée en ce que** l'unité d'aquaculture (2) présente au moins une zone pour la pisciculture (8), un filtre mécanique (9) et un filtre biologique (10).

6. Installation aquaponique (1) selon une des revendications précédentes, **caractérisée en ce que** aussi bien l'unité d'aquaculture (2) que l'unité hydroponique (5) sont disposées dans une serre commune de façon fonctionnelle de sorte qu'il en résulte un espace commun cohérent qui est raccordé au piège à froid (6), au moins au nombre de un, de l'unité hydroponique (5).

7. Installation aquaponique (1) selon une des revendications précédentes, **caractérisée en ce que** l'apport quotidien d'eau fraîche lors du fonctionnement de l'installation représente moins de 5 % du volume d'eau total de l'installation.

8. Installation aquaponique (1) selon une des revendications précédentes, **caractérisée en ce que** l'installation aquaponique (1) présente en outre une installation photovoltaïque.

9. Installation aquaponique (1) selon une des revendications précédentes, **caractérisée en ce que** l'installation aquaponique (1) présente une installation de biogaz.

10. Procédé de fonctionnement d'une installation aquaponique (1), **caractérisé en ce que**
a) de l'eau est conduite depuis une unité d'aquaculture (2) à une unité hydroponique (5) en passant par une évacuation d'eau (3) avec une vanne à une voie (4) ;
b) l'eau est prélevée sur des plantes de l'unité hydroponique (5) et délivrée, par évapotranspiration par les plantes, à l'atmosphère de l'unité hydroponique ;
c) l'eau en provenance de l'atmosphère de l'unité hydroponique (5) est collectée par condensation ; et
d) l'eau collectée est reconduite à l'unité d'aquaculture (2).

11. Procédé selon la revendication 10, **caractérisé en ce que** l'unité d'aquaculture (2) est exploitée avec de la nourriture exempte de farine de poissons et/ou de d'huile de poissons.

12. Procédé selon une des revendications 10 ou 11, **caractérisé en ce que** l'unité d'aquaculture (2) est exploitée avec des tilapias, de préférence avec *Oreochromis niloticus.*

13. Procédé selon une des revendications 10 à 12, **caractérisé en ce que** l'unité hydroponique (5) est exploitée avec des plantes potagères, de préférence avec des tomates et/ou des concombres.

14. Procédé de production de produits d'aquaculture, **caractérisé en ce qu'**une installation aquaponique (1) est mise en oeuvre selon une des revendications 1 à 9.

15. Procédé de production de produits hydroponiques, **caractérisé en ce qu'**une installation aquaponique (1) est mise en oeuvre selon une des revendications 1 à 9.

16. Utilisation d'une installation aquaponique (1) selon une des revendications 1 à 9 pour l'obtention de produits d'aquaculture ou de produits hydroponiques.
